# EUROPÄISCHE PATENTANMELDUNG

(11) **EP 1 317 934 A1**
(43) Veröffentlichungstag der Anmeldung: **11.06.2003**
(21) Anmeldenummer: 01126706.9
(22) Anmeldetag: 08.11.2001
(51) Int. Cl.: A61L 27/36

(54) **Verfahren zur Herstellung einer azellularisierten Gewebematrix und damit erhältliche Gewebematrix**

(71) Anmelder: Artiss GmbH, 30625 Hannover (DE)
(72) Erfinder: Haverich, Axel, Professor Dr., 30916 Isernhagen (DE); Mertsching, Heike, Dr., 31535 Neustadt (DE); Brenning, H. Ole, 30173 Hannover (DE)
(74) Vertreter: Grünecker, Kinkeldey, Stockmair & Schwanhäusser Anwaltssozietät

(57) **Zusammenfassung**

Die Erfindung betrifft ein Verfahren zur Herstellung einer azellularisierten Gewebematrix sowie eine damit erhältliche Gewebematrix, wobei das erfindungsgemäße Verfahren die Schritte umfasst:
a) Bereitstellen eines natürlichen Gewebes,
b) Behandeln des Gewebes mit einer wässrigen Lösung A, die wenigsten ein Natrium- und/oder ein Kaliumsalz umfasst,
c) Behandeln des Gewebes mit einer wässrigen Lösung B), die wenigstens ein Natrium- und/oder ein Kaliumsalz umfasst und deren Salzkonzentration verschieden von der der wässrigen Lösung A) ist, und
d) mechanische Entfernung der Zellen.

## Beschreibung

Die Erfindung betrifft ein Verfahren zur Herstellung einer azellularisierten Gewebematrix sowie eine damit erhältliche Gewebematrix.

Weltweit besteht ein großer Bedarf an Transplantaten und biologischen Implantaten. Dazu können menschliche Spenderorgane (Allotransplantate), tierische Spenderorgane (Xenotransplantate) oder bioartifizielle Gewebe verwendet werden. Wegen der zu geringen Verfügbarkeit von Allo- und Xenotransplantaten hat die Herstellung von bioartifiziellen Geweben in den letzten Jahren zunehmend an Bedeutung gewonnen.

Dazu wird ein gewünschtes Gewebe einem Spender entnommen, azellularisiert und mit Zellen wiederbesiedelt, um ein bioartifizielles Gewebe zu erhalten. Wenn das Gewebe einem vom Empfänger verschiedenen Spender entnommen wird, ist es wichtig, dass die Azellularisierung vollständig und sicher durchgeführt wird, um spätere Probleme bei der Implantation des bioartifiziellen Gewebes, wie Fremdkörperreaktionen, Abstoßungsreaktionen, oder Tumorgenese im Empfänger, zu verhindern. Diese Probleme wurden herkömmlich durch lebenslange Immunsuppression oder eine Denaturierung des Gewebes durch Kryokonservierung versucht zu vermindern.

Verfahren zur Azellularisierung eines Gewebes sind bekannt, wie das Ablösen der Zellen durch eine Inkubation in einer rein wässrigen Lösung ohne Salze oder der enzymatische Abbau der Zellen durch eine Inkubation in einer Trypsin/EDTA-Lösung. Die Verfahren sind langwierig und dauern bis zu 4 Tage.

Die herkömmlichen Verfahren haben außerdem den Nachteil, dass die vorhandenen Zellen oftmals nicht vollständig abgebaut werden und sich durch die notwendig langen Inkubationszeiten in wässrigen Lösungen Wasser in die Collagenstrukturen einlagert, wodurch es zur Quellung der Matrix kommt. Diese aufgequollenen Matrixstrukturen eignen sich nicht mehr oder nur sehr schlecht zur gewebespezifischen Rebesiedlung. Die resultierenden bioartifiziellen Gewebe sind wesentlich instabiler als die ursprünglichen (nativen) Gewebe.

Daher liegt der vorliegenden Erfindung die Aufgabe zugrunde, ein einfacheres, schnelleres und schonenderes Verfahren zur Herstellung einer Gewebematrix zur Verfügung zu stellen.

Die Lösung der Aufgaben ist ein Verfahren, umfassend die Schritte
a) Bereitstellen eines natürlichen Gewebes,
b) Behandeln des Gewebes mit einer wässrigen Lösung A), die wenigstens ein Natrium- und/oder ein Kaliumsalz umfasst,
c) Behandeln des Gewebes mit einer wässrigen Lösung B), die wenigstens ein Natrium- und/oder ein Kaliumsalz umfasst und deren Salzkonzentration verschieden von der der Lösung A) ist, und
d) mechanische Entfernung der Zellen.

Zur Bereitstellung eines natürlichen Gewebes in Schritt a) kann jedes, eine Gewebematrix enthaltende Gewebe verwendet werden, das eine für die Herstellung eines Transplantats gewünschte Anordnung hat. Das Gewebe kann xenogen, allogen oder autolog in Bezug auf den Transplantatempfänger sein. Vorzugsweise können dafür Hohlorgane, Blutgefäße, wie Aorta oder Vena Cava, Gewebe aus dem Uro-Genital-Trakt, z.B. Blase, Harnröhre, Harnleiter, und Gewebe aus dem Gastro-Intestinal-Trakt, wie die submukosale Schicht der Darmwand oder Teilbereiche der Speiseröhre, des Magens, Jejunums, lleums oder Colons verwendet werden. Insbesondere bevorzugt kann ein Gewebe mit einem daran befindlichen Blutgefäß verwendet werden, um die Versorgung des Gewebes und das Einwachsen des Gewebes an dem späteren Empfänger zu gewährleisten. Insbesondere bevorzugt wird ein Gewebe mit einem daran befindlichen arteriellen und einem daran befindlichen venösem Gefäß in Schritt a) bereitgestellt. Als Spender für das Gewebe kommen Menschen oder Tiere in Betracht, insbesondere Schweine. Bevorzugt stammt das Gewebe von dem späteren Implantatempfänger.

Das Gewebe kann zunächst mit gekühlter, bevorzugt etwa 4°C kalter, NaCI-Lösung, die etwa 0,9 %ig sein kann, perfundiert und sodann in ebenfalls gekühlter, bevorzugt etwa 4°C kalter, Nebacetin-NaCI-Lösung transportiert und gelagert werden.

Das bereitgestellt Gewebe wird in Schritt b) mit einer wässrigen Lösung A) behandelt, die wenigstens ein Natrium- und/oder Kaliumsalz umfasst. Vorzugsweise enthält die wässrige Lösung A) wenigstens zwei Natrium- und zwei Kaliumsalze. Insbesondere bevorzugt enthält die wässrige Lösung A) NaCI, Na-Citrat, KH₂PO₄ und KCI. Die Salzkonzentration der wässrigen Lösung A) ist vorzugsweise etwa 100 bis 150 mM, insbesondere etwa 130 mM. Der pH-Wert der wässrigen Lösung A) liegt vorzugsweise im Bereich von 6 bis 7,5, insbesondere von 7 bis 7,3.

Vorzugsweise wird in Schritt b) der Lumeninhalt des bereitgestellten Gewebes mit wässriger Lösung A) befüllt und sodann in einer salzhaltigen-Lösung, vorzugsweise einer NaCI-Lösung, inkubiert. Die NaCI-Lösung ist vorzugsweise etwa 1 bis etwa 10%ig, bevorzugter etwa 1 bis etwa 3 %ig, insbesondere etwa 1,32 %ig, und hat vorzugsweise ein Temperatur von etwa 37 °C im Wasserbad und die Inkubation kann beliebig lange durchgeführt werden, wird jedoch vorzugsweise bis zu etwa 3 Std., bevorzugter bis zu etwa 1 Std. und insbesondere bis zu etwa 40 min. durchgeführt. Die Inkubation kann auch länger durchgeführt werden, jedoch wird ein befriedigendes Ergebnis bereits bei einer Inkubation von bis zu etwa 40 min. erreicht.

Sodann kann der Lumeninhalt abgelassen und ausmassiert werden.

Danach wird das Gewebe in Schritt c) mit einer wässrigen Lösung B) behandelt, die wenigstens ein Natrium- und/oder ein Kaliumsalz umfasst und deren Salzkonzentration verschieden von der der wässrigen Lösung A) ist. Es ist bevorzugt, dass die Salzkonzentration höher ist, als die der Lösung A). Insbesondere ist es bevorzugt, dass die Salzkonzentration der Lösung B) 1,5 - 3 -fach höher ist, als die der Lösung A). Vorzugsweise enthält die wässrige Lösung B) mindestens zwei Natrium- und zwei Kaliumsalze. Insbesondere bevorzugt enthält die wässrige Lösung B) NaCI, Na₂HPO₄, KH₂PO₄ und KCI. Die Konzentration der Komponenten ist vorzugsweise etwa 130 bis 180 mM, insbesondere etwa 150 mM. Der pH-Wert der wässrigen Lösung B) liegt vorzugsweise im Bereich von etwa 6 bis 7,5, insbesondere von 7 bis 7,4. Der pH-Wert der wässrigen Lösung B) ist vorzugsweise höher als der der wässrigen Lösung A). Außerdem kann die wässrige Lösung B) Komplexbildner, wie EDTA, enthalten.

Vorzugsweise wird in Schritt c) der Lumeninhalt des Gewebes mit wässriger Lösung B) befüllt und, wie vorstehend für Schritt b) beschrieben, in einer salzhaltigen-Lösung, vorzugsweise einer NaCI-Lösung, inkubiert. Die Dauer der Inkubation ist ebenfalls, wie vorstehend für Schritt b) beschrieben.

Anschließend kann der Lumeninhalt abgelassen und ausmassiert werden. Durch die Schritte b) und c) wird die innere Zellschicht des Gewebes entfernt.

Schritt c) des erfindungsgemäßen Verfahrens kann auch vor Schritt b) durchgeführt werden, es ist jedoch bevorzugt, die Schritte b) und c) in der angegebenen Reihenfolge durchzuführen.

In Schritt d) werden die Zellen mechanisch entfernt. Durch die mechanische Entfernung wird die Gewebematrix vor schädigenden Einflüssen chemischer Substanzen bewahrt, wodurch ein besonders schonendes Verfahren erhalten wird. Die Collagenstruktur und -beschaffenheit (z.B. Anwesenheit von Wachstumsfaktoren) bleibt erhalten. Man erhält eine erfindungsgemäße Gewebematrix.

Falls das in Schritt a) bereitgestellte Gewebe von einem allogenen oder xenogenen Spender stammt, ist es bevorzugt, nach dem Entfernen der Zellen die erhaltene Gewebematrix in eine Durchflußvorrichtung einzubringen und bei starkem Fluß (z.B. 100 U/min) durch Druck und Reibung den verbliebenen Zellschrott zu entfernen. Vorzugsweise wird dabei durch intra- und / oder extraluminales Spülen mit einer Lösung C), die vorzugsweise Natriumdeoxycholat und Natriumazid enthält, der Zellschrott entfernt und das Gewebe vollständig azellularisiert. Die Behandlung erfolgt bevorzugt durch etwa einstündiges Inkubieren bei etwa 4°C. Die Inkubation kann auch länger durchgeführt werden, jedoch werden befriedigende Ergebnisse bereits bei einer etwa einstündigen Inkubation erreicht.

Anschließend können die entstanden Zelltrümmer, insbesondere durch Perfusion mit salzhaltigen-Lösung, vorzugsweise PBS (z.B. 100U/min) entfernt werden und die Lösung C) durch Inkubieren bei 37°C inaktiviert werden.

In dem erfindungsgemäßen Verfahren können die Schritte b) bis d) in jeder beliebigen Reihenfolge durchgeführt werden. Es ist jedoch bevorzugt, die Schritte in angegebenen Reihenfolge durchzuführen.

Die so erhaltene erfindungsgemäße Gewebematrix kann dann mit üblichen Verfahren mit Zellen besiedelt werden, wodurch ein bioartifizielles Gewebe, das dem geeigneten Empfänger eingesetzt werden kann, erhalten wird.

In Abhängigkeit von für die Besiedelung verwendetem Zelltyp kann die erfindungsgemäße Gewebematrix vor der Besiedelung etwa 24 Stunden bei 37°C im Zellkulturmedium vorinkubiert werden.

Das erfindungsgemäße Verfahren hat den Vorteil, dass es zeitsparend und schonend ist.

So kann das Verfahren erfindungsgemäß innerhalb von 80 bis 140 min (40 min für Schritt b), 40 min für Schritt c) und 60 min für Schritt d)) durchgeführt werden. Durch die drastische Zeitersparnis im Herstellungsprozeß einer Gewebematrix wird die Einwirkdauer von chemischen Substanzen oder Lösungen minimiert. D.h., die Matrix bleibt in ihrer Struktur und Beschaffenheit erhalten. Es kommt z.B. zu einer minimalen Wassereinlagerung in dem Kollagengerüst. Die Matrix muß nicht zwingend chemischen Substanzen ausgesetzt werden, die eine spätere Rebesiedelung mit Zellen negativ beeinflussen oder unmöglich machen könnten, und die native Kollagenfaserstrukturen und deren Inhaltsstoffe können nahezu unbeschädigt erhalten bleiben.

Die Verfügbarkeit des bioartifiziellen Gewebes oder Organs wird beschleunigt. Somit könnten in Zukunft benötigte Gewebe oder lebensrettende Organe bis nahezu 96 Stunden früher zur Verfügung stehen und die mit dem erfindungsgemäßen Verfahren erhaltenen bioartifiziellen Gewebe/Organe sind bereits bei Implantation mechanisch belastbar.

Die mit dem Verfahren hergestellte erfindungsgemäße Gewebematrix hat gegenüber herkömmlich verwendeten Matrices den Vorteil , dass der Herstellungsprozeß bis zu 96 Stunden kürzer ist, so dass das Grundgewebe diesen Zeitraum betreffend kürzer chemischen Substanzen ausgesetzt ist. Somit wird die Struktur der zu erschaffenen Matrix kürzer unphysiologischen Einflüssen ausgesetzt und nach kürzester Zeit steht eine Matrix zur erfolgreichen Rebesiedelung zur Verfügung.

Die erfindungsgemäße Gewebematrix ist außerdem mechanisch stabiler als herkömmliche Matrices. Damit ist die Stabilität z.B. im pulsierendem System, das Nahtverhalten und die Langzeitfunktion der unter Verwendung der erfindungsgemäßen Matrix erhaltenen Transplantate, z. B. im arteriellen Bereich, wie Herzklappen und arteriellen Gefäßen, verbessert.

Die Erfindung wird nachstehend anhand von Beispielen erläutert.

### Beispiel 1

Ein Teilbereich des Magen-Darm-Trakts mit arteriellem Gefäßast und intaktem intramuralem Kapillarnetz, der mit 4 °C kalter NaCI 0,9%iger Lösung perfundiert war, wurde entnommen. Während des Transports und der Lagerung wurde das Gewebe in einer 4 °C kalten Nebacitin-NaCI-Lösung (1g Nebacitin/1L NaCl) eingelegt. Sodann wurde die Mucosa durch Befüllen des Lumens mit einer Lösung A und einer 40 minütigen Inkubation in einer 1,32 %igen NaCI-Lösung bei 37 °C im Wasserbad entfernt. Anschließend wurde der Lumeninhalt abgelassen und ausmassiert. Das Lumen wurde erneut mit einer Lösung B befüllt und wiederum bei 37 °C im Wasserbad inkubiert. Danach wurde der Lumeninhalt ausgelassen und ausmassiert.

| Lösung A | 96 mM NaCI |
|---|---|
| | 27 mM Na-Citrat x 2 H₂O |
| | 5,6 mM KH₂PO₄ |
| | 1,5 mM KCI |
| | |
| | pH 7,3 |

| Lösung B | 137,0 mM NaCl |
|---|---|
| | 8,2 mM Na₂HPO₄ |
| | 1,5 mM KH₂PO₄ |
| | 3,2 mM KCI |
| | 1,5 mM EDTA |
| | pH 7,4 |

Anschließend wurde die Muskularis mechanisch entfernt und das erhaltene Gewebe in eine Durchflußvorrichtung eingebracht. Der Abbau der verbleibenden Zellen wurde mit einer intra- und extra-luminalen Füllung des Darms mit einer Lösung C und einer einstündigen Inkubation bei 4 °C durchgeführt. Die Zelltrümmer und die verbleibende Lösung C wurden durch Perfusion (100 U/min) mit PBS entfernt. Danach wurde bei 37 °C inkubiert, um möglicherweise verbliebene Rückstände der Lösung C zu inaktivieren. Dadurch wird eine azellularisierte Gewebematrix erhalten.

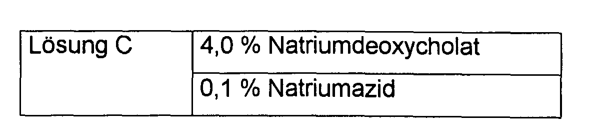

### Beispiel 2

Ein Teilbereich des Magen-Darm-Trakts mit einem arteriellen, einem venösen Gefäßast und intaktem intramuralem Kapillametz wurde gemäß Beispiel 2 behandelt. Man erhält eine erfindungsgemäße Gewebematrix.

## Patentansprüche

1. Verfahren zur Herstellung einer Gewebematrix, umfassend die Schritte
a) Bereitstellen eines natürlichen Gewebes,
b) Behandeln des Gewebes mit einer wässrigen Lösung A, die wenigsten ein Natrium- und/oder ein Kaliumsalz umfasst,
c) Behandeln des Gewebes mit einer wässrigen Lösung B), die wenigstens ein Natrium- und/oder ein Kaliumsalz umfasst und deren Salzkonzentration verschieden von der der wässrigen Lösung A) ist, und
d) mechanische Entfernung der Zellen.

2. Verfahren nach Anspruch 1, wobei die Salzkonzentration der in Schritt c) verwendeten Lösung B) höher ist, als die der in Schritt b) verwendeten Lösung A).

3. Verfahren nach einem der vorstehenden Ansprüche, wobei die wässrigen Lösungen A) und/oder B) mindestens zwei Natrium- und Kaliumsalze umfassen.

4. Verfahren nach einem der vorstehenden Ansprüche, wobei die in Schritt b) verwendete wässrige Lösung A) NaCI, NaCitrat, KH₂PO₄ und KCI enthält.

5. Verfahren nach einem der vorstehenden Ansprüche, wobei die in Schritt c) verwendete wässrige Lösung B) NaCI, Na₂HPO₄, KH₂PO₄ und KCI enthält.

6. Verfahren nach einem der vorstehenden Ansprüche, wobei das Gewebe vor dem Durchführen des Verfahrens in gekühlter wässriger NaCIhaltiger Lösung gelagert wird.

7. Verfahren nach einem der vorstehenden Ansprüche, wobei vor, während oder nach Schritt d) die Zellen zusätzlich chemisch entfernt werden.

8. Verfahren nach Anspruch 7, wobei die chemische Entfernung der Zellen mit einer Lösung C) durchgeführt wird, die Natriumdeoxycholat und Natriumazid enthält.

9. Gewebematrix, erhältlich durch ein Verfahren nach einem der Ansprüche 1 bis 8.
